# EUROPEAN PATENT SPECIFICATION

(11) **EP 0 594 803 B1**
(45) Date of publication and mention of the grant of the patent: **29.10.2003**
(21) Application number: 93907459.7
(22) Date of filing: 18.03.1993
(51) Int. Cl.: A61K 9/127, A61K 9/133, A61K 9/64, A61K 31/195, A61K 31/505, A61K 38/44, A61K 38/46, A61K 39/44, C12N 9/96, C12N 11/02, A61K 47/48

(54) **COMPOSITION FOR REDUCING SIDE-EFFECTS OF A DRUG**
ZUSAMMENSETZUNG ZUR VERMINDERUNG VON NEBENWIRKUNGEN EINES ARZNEIMITTELS
COMPOSITION PERMETTANT DE REDUIRE LES EFFETS SECONDAIRES D'UN MEDICAMENT

(30) Priority: 23.03.1992 US 855586
(43) Date of publication of application: 04.05.1994
(73) Proprietor: MATSUMURA, Kenneth Naoyuki, Berkeley, CA 94704-2062 (US)
(72) Inventor: MATSUMURA, Kenneth Naoyuki, Berkeley, CA 94704-2062 (US)
(74) Representative: Davies, Gregory Mark
(86) International application number: US9302272
(87) International publication number: WO93018750

(56) References cited:
- EP-A- 0 173 145
- EP-A- 0 279 862
- WO-A-90/10460
- WO-A-91/17761
- US-A- 4 289 872
- US-A- 4 659 655
- US-A- 4 957 735
- CHEMICAL ABSTRACTS, vol. 117, no. 3, 20 July 1992, Columbus, Ohio, US; abstract no. 21595, & METALLOTHIONEIN BIOL. MED., 1991 pages 305 - 310 NAGNUMA AKIRA ET AL. 'EFFECT OF PREINDUCTION OF METALLOTHIONEIN SYNTHESIS ON TOXICITY OF FREE RADICAL GENERATING COMPOUNDS IN THE MOUSE.'
- CHEMICAL ABSTRACTS, vol. 102, no. 24, 17 June 1985, Columbus, Ohio, US; abstract no. 209350, & METHODOL. SURV. BIOCHEM. ANAL., vol.13, 1984 pages 331 - 336 ISOM GARY E. ET AL. 'TARGETED DRUG CARRIERS: BIOLOGICAL ACTIVITY OF THE N-ACETYLCYSTEINE-LIPOSOME SYSTEM.'
- DATABASE BIOSIS BIOSCIENCES INFORMATION SERVICE, PHILADELPHIA, PA, US AN=92:461543 & JPN J. TOXICOL. ENVIRON.HEALTH, vol.38, no.3, 1992 pages 228 - 239 SATO M. 'BIOLOGICAL ANTIOXIDANT DEFENSE SYSTEM AND METALLOTHIOEIN.'
- CANCER CHEMOTHERAPY AND PHARMACOLOGY, vol.21, 1988 pages 176 - 178 MASHIKO SATOH ET AL. 'METALLOTHIONEIN INDUCTION PREVENTS TOXIC SIDE EFFECTS OF CISPLATIN AND ADRIAMYCIN USED IN COMBINATION.'
- ARCH. OF TOXICOLOGY, vol.66, 1992 pages 145 - 148 MASAHIKO SATOH ET AL. 'EFFECT OF PREINDUCTION OF METALLOTHIONEIN ON PARAQUAT TOXICITY IN MICE.'
- Cancer Chemotherapy and Pharmacology, Volume 26, issued 1990, V. KEEGAN-ROGERS et al., "Targeted protection of hepatocytes from galactosamine toxicity in vivo", pages 93-96, especially pages 93-95.
- The Journal of Biological Chemistry, Volume 263, No. 10, issued 05 April 1988, G.Y. WU et al., "Targeted Antagonism of Galactosamine Toxicity in Normal Rat Hepatocytes in vitro", pages 4719-4723, especially pages 420-423.
- Hepatology, Volume 5, No. 5, issued 1985, G.Y. WU et al., "Acetaminophen Hepatotoxicity and Targeted Rescue: A Model for Specific Chemotherapy of Hepatocellular Carcinoma", pages 709-713, especially pages 711 and 712.
- Proc. Natl. Acad. Sci. USA, Volume 80, issued May 1983, G.Y. WU et al., "Model for specific rescue of normal hepatocytes during methotrexate treatment of hepatic malignancy", pages 3078-3080, especially pages 3078 and 3079.
- Methods in Enzymology, Volume 44, issued 1976, G. GREGORIADIS, "Medical Applications of Liposome-Entrapped Enzymes", pages 698-709, especially pages 699,700 and 704.

## Description

### Field of Invention

This invention is in the field of chemotherapy. More specifically, it deals with compositions of reducing side effects of chemotherapeutic agents.

### Background of Invention

Drugs could be made more effective if side effects did not prevent their use in stronger dosages. Side effects are caused by their action not being sufficiently focussed on the body cells or microorganisms needing to be treated. The side effects are specifically caused by misdirected action of said drug on certain body cells which are not the intended target of said drug. For example, if the drug intended for other targets is nevertheless toxic to gastrointestinal cells, then nausea, vomiting, and diarrhea, as well as gastrointestinal bleeding can result. If the drug is toxic to hematopoietic (blood making) cells, anemia, susceptibility to infection (from insufficient number of white corpuscles), and bleeding can result. If the drug is toxic to cells of the skin, hair loss and rash can result.

The reason cancer is so difficult to eradicate from the body is that agents (hereinafter referred to as oncolytics or anti-cancer drugs) which kill cancer cells also kill normal dividing cells like those of the intestine, hematopoietic bone marrow, and the skin. If normal cells can be protected from the cytotoxic effects of anti-cancer agents, it would become safe to use higher doses of the anti-cancer agents. Since higher doses kill a higher percentage of cancer cells, it would become possible to kill those few cancer cells able to survive the lower dosages currently in use.

The reason viruses are so difficult to eradicate from the body is that agents which kill viruses also kill normal dividing cells. If normal cells can be protected from the cytotoxic effects of anti-viral agents, we would be able to use sufficient dosages of such agents to eradicate life-threatening viruses. For example, azidothymidine (AZT) has been found to be life-prolonging in the treatment of Acquired Immune Deficiency Syndrome (AIDS). According to Backgrounder (published by the National Cancer Institute, Office of Cancer Communications), September 19, 1986, "AZT is a derivative of thymidine, one of the normal components of DNA (genes), which is needed by the host cell and the virus to make chemicals necessary for replication. When AZT enters a cell infected by HTLV-III (the virus that causes AIDS), the drug floods the cell with false DNA building blocks so the virus cannot make copies of itself. Once this happens, viral infection and replication is halted, thereby protecting target cells." Unfortunately, azidothymidine injected into the blood stream also reaches hematopoietic cells of bone marrow. There, azidothymidine halts replication of reproducing blood cells. The result is anemia and reduced white blood cell count. The NCI Backgrounder (just cited) states, "at high doses, it is likely that bone marrow suppression will be a limiting factor for the drug." If we can protect these bone marrow cells from the harmful action of AZT, we would be able to use a higher dosage of AZT possibly leading to better treatments. While there are now substantial questions whether AIDS-associated virus is the cause of AIDS, the foregoing description would remain valid for the treatment of viral diseases in general where protection of normal dividing cells allows higher, more effective dosages of antiviral agents.

Other anti-microbial drugs have serious side effects that limit their use. Gentamycin and related drugs like tobramycin and amikacin are, next to penicillin, some of the most important antibiotics in the treatment of sepsis in debilitated patients (e.g., the post-surgical, the elderly, the immunity-compromised). These drugs, however, also damage kidney cells as well as vestibular and auditory sensory cells and can not often be used in adequate dosages or for sufficient duration. Protecting the kidney, vestibular and auditory sensory cells from the harmful action of these antibiotics can enable more prolonged use of these drugs that can lead to saving of more lives.

WO-A-9 010 460 is generally concerned with a method for reducing side effects of a drug caused by undesired effects of said drug upon body cells, which are not the intended target of said drug. From this document it is known that antibodies are needed to direct a microbody carrier containing an antidote to a certain body cell. However, passive directing compositions, consisting of liposomes and antidote, are not disclosed.

WO-A-9 117 761 discloses a method for targeted protection from cytotoxines, wherein an antagonist of the cytotoxin complexed with a cell-specific binding agent, which specifically binds a cellular surface component present on normal, but not diseased cells, is used for treatment. However, this disclosure does not show a composition for the delivery of an antidote to a body cell, which composition is absorbed by said body cell by its inherent affinity.

Chemical Abstracts, Vol. 102, No. 24 (1985), abstract No. 209350 discloses drug carriers designed to selectively deliver a pharmacologic agent to the site of action in order to increase efficacy and minimize side-effects of the agent itself. This document talks about liposomes that are unilamellar, but 200 to 300 nm in size. However, larger liposomes (200 to 300 nm) tend to be cleared out of the bloodstream by phagocytic macrophage cells and by the liver. Therefore, the liposomes that ire disclosed will not work to solve the problems of the present invention.

### Objects & Summary of the Invention

I have invented a novel composition for focussing of drug effect and reducing side effects of a drug by protecting body cells not meant to be affected by said drug from the unwanted effects of said drug. More specifically, my composition is to preferentially deliver to the body cells needing said protection an antidote for said drug when said drug is used. Said delivery being accomplished so as to enable said antidote to counteract undesirable actions of said drug on said body cells needing protection and to do so on said body cells needing protection in preference over the intended target of said drug.

In a preferred embodiment, my composition is to preferentially deliver to the body cells needing said protection an antidote for said drug when said drug is used, said preferential delivery being accomplished by placing said antidote on a small, soft-shelled unilamellar liposome with an inherent affinity to be taken up by said cells needing protection, said affinity for uptake being greater by said cells needing protection than by the intended target of said drug, said uptake enabling said antidote to counteract undesirable actions of said drug on said body cells needing protection.

### Detailed Description of the Invention

By the term "drug", I mean chemotherapeutic agents such as those listed in The Pharmacologic Basis of Therapeutics (A.G. Gilman, L.S. Goodman, A. Gilman, editors, McMillan Publishing Co. New York 1980 and later editions). For this invention, the term "drug" includes ionizing radiation when used in treatment of diseases since the radioactive high energy particles act as drugs.

By the term "antidote", I mean a chemical that counteracts the cellular effect of a specific drug. For example, folinic acid is an antidote for anti-cancer drug methotrexate, thymidine is an antidote for anti-cancer drug floxuridine (FUdR), oxypurinol is an antidote for 5-fluorouracil (5-FU), uridine is an antidote for azaribine, thiol containing chemicals such as thiourea, methionine (Burchenel, J.H. et al. Biochimie 60: 961-965, 1978; DiRe, F. et al. Cancer Chemother. Pharm. 25: 355-360, 1990) are antidotes for platinum and platinum derivatives, and deoxycytidine is an antidote for cytosine arabinoside. Folinic acid is a weak antidote for vinblastine and vincristine, but glutamic and aspartic acids, sodium glutamate land tryptophan are antidotes for vinblastine.

There is a certain logic about determining what chemical would serve as an antidote for any given drug:
1) If the drug acts by being a non-functional analog of a natural metabolite (for example, the enzyme dihydrofolate reductase is blocked by methotrexate, a non-functional analog of folinic acid, in the production of thymidine), one antidote for such a drug is the natural (functional) metabolite for which this drug is the analog (for methotrexate, folinic acid, which can compete for the enzyme binding site to prevent methotrexate from binding and blocking the enzyme).
2) If the drug acts by reversibly blocking an enzyme critically needed for the production of a specific metabolite (for example, the enzyme dihydrofolate reductase is blocked by methotrexate in the production of thymidine), one antidote for such a drug is the specific metabolite whose production is being blocked by the drug (for example, in the case of methotrexate, thymidine) ;
3) If natural resistance to a given drug is found to develop in cells, one can learn how cells become resistant and find a chemical which can make the cell resistant to the drug in the same manner; such a chemical could be an antidote. For example, cells often become more resistant to the action of a drug by increasing the synthesis of an enzyme that can help degrade the drug once the drug enters the cell. Said enzyme is an antidote for the drug. As a corollary to this rule, an enzyme that can function otherwise harmlessly in the intracellular milieu which degrades the drug can be an antidote. Some cells are resistant to the action of bleomycin because of the higher intracellular concentration of bleomycin hydrolase. Resistance to cytosine arabinoside has also been attributed to cytidine deaminase. Resistance to important aminoglycoside antibiotics has been attributed to acetylases and aminoglycoside-inactivating enzymes. Xanthine oxidase found plentifully in the liver detoxifies 6-mercaptopurine and azathioprine. These enzymes, when delivered into the cytoplasm of cells needing protection, are antidotes for the drugs they detoxify.
4) For ionizing radiation as a drug, one can find antidotes among chemicals known to be "radioprotectors", such as WR-2721 and WR-1065 (Walter Reed Army Institute of Research, Washington, D.C.).
5) A new approach I am still exploring is to deliver into the cellular cytoplasm an exogenous excess of the target structures to which the drug binds (cisplatin to deoxyribonucleic acid), intercalates with (doxorubicin, daunorubicin with deoxyribonucleic acid), or directly alters (alkylators with DNA and components like guanine). In this case, fragments of DNA (e.g., guanine-base rich DNA fragments, artificially synthesized) would be the antidote. Once inside the cytoplasm, such decoy DNA fragments could react with entering cytotoxic drug molecules rendering them harmless to the "real" functioning DNA of the cell.
6) Some drugs act via generation of free-radicals inside cells (for example, doxorubicin). In such cases, free-radical scavengers like alpha-tocopherol, coenzyme Q, and N-acyl dehydroalanines can serve as antidotes (Solaini, G. Biochem. Biophys. Res. Comm. 147 (2): 572-80. 1987 & its references; Pascoe, G.A. Archives Bioch. Biophys. 256(1): 159-166, 1987). Doxorubicin's use is limited by its cardiotoxicity which appears to be particularly caused by free-radical generation. Aforementioned scanvengers can serve as antidotes to the heart muscle and other cells. Metallothionein (Webb, M. In: The chemistry, biochemistry and biology of cadmium, Webb, M., ed. Elsevier/North Holland, Amsterdam, p. 195, 1979) is a low molecular weight protein exhibiting a protective action against heavy metal toxicity, such as of platinum drugs, and also appears to have protective effect on cells against free-radical forming drugs, such as doxorubicin, bleomycin, peplomycin, and irradiation. Metallothionein has also been reported as protective against some alkylating drugs. One can use metallothionein directly as an antidote, or use a chemical, such as bismuth subnitrate which pre-induces the synthesis within cells of metallothionein (Satoh, M, et al. Cancer Chemother. Pharmacol. 21: 176-178, 1988). It would of course be common sense, and certainly obvious to those skilled in the art, that if one uses a pre-inducer, one would optimize the protective effect if one delivers the pre-inducer in advance of the use of the cancer drug so that there would be time for the metallothionein to be formed in the cell to be protected before the cell is exposed to the cancer drug. However, since cancer drugs are typically applied to patients repeatedly over many weeks, one can begin the pre-inducer treatment around the same time as the cancer drug. At first, the cells to be protected are not optimally protected, but within a few days, said cells will be optimally protected.

When seeking antidotes, one can use known empirical evidence or conduct in vivo or in vitro tests. For example, one can determine if a substance qualifies as an "antidote" for the purposes of this invention as follows: one introduces the test "antidote" into a tissue culture of body cells needing protection. Then the drug for which the antidote is being tested is introduced into the tissue culture. One assesses the effect that the test "antidote" has on the action of the drug on the cells in tissue culture so that one can determine the acceptability of the test "antidote". For some antidotes, like proteins and enzymes, one may need to provide means for the test antidote to get inside the cultured body cells (for example, by placing the enzymes inside liposomes (discussed below) and allowing the enzymes to enter the cytoplasm when the membranes of the liposomes and the cell fuse.

Liposomes are spherules formed when phospholipids are allowed to swell in aqueous compartments. Within the lipid or aqueous phase of liposomes, lipid or watersoluble substances, respectively, can be entrapped. Several of physical properties of liposomes can be varied at will: size (radius) can be adjusted from about 12 nm for unilamellar liposomes to up to several microns for the multilamellar versions, and a negative or positive surface charge can be imposed by the incorporation of charged amphiles (Gregoriadis, G., Methods in Enzymology 44: 698-, 1976) Control of permeability to entrapped substances, and of stability is also feasible by the addition of a sterol or other lipids into the liposomal structure (Gregoriadis, G. reference just cited).

Preferably, the liposome should be unilamellar, about 35 to 60 nm (350 to 600 angstroms) in diameter and either neutral or positively charged. Many methods are well-known in the art for making suitable liposomes (Juliano, R.L., Stamp, D., Biochem. Biophys. Res. Comm. 63 (3): 651-658, 1975; also Ann. New York Acad. Sci. 308: 411-432, 1978, also Canad. J. Physio. Pharmacy 57 (5): 535-539, 1979, and Biochem. Pharmacol. 27: 21-27, 1978; Kimelberg H.K., Cancer Res. 36: 2949-2957, 1976, work of Barbet, J., Machy, P., & L. Leserman, J. Supramolecular Structure and Cellular Biochemistry 16: 243-258, 1981; Gregoriadis, G. Nature 265: 407-11, 1977; Gregoriadis, G., New England J. Med. 295 (3): 704-710, 1976 and 295 (14): 765-770, 1976). Plentiful information is available in the research literature that describes how to vary the size, charge and content of the liposomes (Gregoriadis, G., Leathwood, P.D., & Ryman, B.E., Fed. Europ. Biochem. Soc. Letters 14: 95-99, 1971; Gregoriadis, G., Fed. Europ. Biochem. Soc. Trans. 2: 117-119, 1974; Gregoriadis, G. & Ryman, B.E., Europ. J. Biochem. 24: 485-491, 1972; Magee, E.E., Miller, O.V., Nature 235: 339-340, 1972; Kobayashi, T., Gann 66: 719-720, 1975; Gregoriadis, G. Biochem. Soc. Trans. 2: 117, 1974; Gregoriadis, G. Fed. Europ. Bioch. Soc. Letters 36 (3) : 292-, 1973; Gregoriadis, G. & E.D. Neerunjun Biochem. Biophys. Res. Comm. 65: 537-544, 1975).

Depending on the type, chemistry, and physical characteristics of the liposome, placing the antidote in such liposome can be advantageous in that some liposomes possess an inherent affinity to be taken up by certain body cells, such as the hematopoietic cells in the bone marrow. One reason for this affinity is that some blood and bone marrow cells have a natural tendency to "engulf" a particulate matter and a microbody carrier constitutes such a particulate matter. In such cases, one can preferentially deliver the antidote to the normal cells needing protection over the drug's intended target merely by designing, developing, or selecting the liposome with the appropriate affinity.

For example, by modifying the structure and the chemical composition of the liposomes, I have discovered that one can direct the liposomes to different cells in the body, for example, the bone marrow cells or the liver cells. Since many drugs, especially oncolytics, affect bone marrow and liver cells detrimentally, one can take advantage of my discovery to deliver the antidotes to the aforementioned normal cells.

Through several years of intense experimentation, I have learned that certain types of liposomes have advantages over other types for the purposes of this invention. Liposomes can be made largely of lipids which are either "fluidy," (liquid-crystalline state) or "solid" at the body temperature. I will hereinafter refer to the fluidy liposomes as being "soft-shelled," and "solid" liposomes as being "hard-shelled," as others have also done. Examples of soft-shelled liposomes are those made of egg phosphatidylcholine (<0) and brain phosphatidylserine (13), numbers in parenthesis being their solid to liquid-crystalline transition temperature in degrees Centigrade. Examples of hard-shelled liposomes are those made of distearoylphosphatidylglycerol (53,7) and distearoylphosphatidylcholine (55,0), numbers in parenthesis again being their solid to liquid-crystalline transition temperatures which are all above the body temperature of 37-40°C. There are also lipids whose solid to liquid-crystalline transition temperatures are close to the body temperature such that liposomes made from them fall somewhere in between being soft and hard-shelled, and can be referred to as being "intermediate hard-shelled." These lipids are: dipalmitoylphosphatidylglycerol (40,0) and dipalmitoylphosphatidylcholine (41,5). Most unexpectedly, I discovered that soft-shelled liposomes possess a greater inherent ability to deliver the antidote to bone marrow cells and counteract the drug toxicity when compared to hard-shelled liposomes. Intermediate hard-shelled liposomes were somewhere in between. Therefore, according to the the present invention, the antidote is placed within small, softshelled unilamellar liposomes that are preferably of 12 to 95 nm (120 to 950 Angstroms) in diameter, made of egg phosphatidylcholine and cholesterol, in the molar ratio of 65:35, respectively) when the goal is to protect the bone marrow cells from toxic cancer drugs. Soft-shelled liposomes also possess greater ability to deliver the antidote to hepatocytes.

The dosage of the antidote delivered to cells needing protection must be adequate to provide said protection. For example, in using folinic acid, one must deliver to the cell being protected at least one mole of folinic acid for each mole of methotrexate entering said cell. There may be advantages in injecting liposomes directly into the arteries feeding the cells needing protection (e.g. coeliac and superior mesenteric arteries feeding gastrointestinal cells).

The antidote should be introduced into the actively circulating body fluid (blood) bathing the cells needing protection before the cytotoxic drug is introduced into the same medium so as to allow the antidote time to reach its target cells. While an antidote like folinic acid has been found to attenuate the toxic effects of methotrexate even when administered 3 hours following methotrexate, attenuation is greater sooner one administers the antidote. In the case of this invention, the antidote preferably would be administered from two to twelve hours before the cytotoxic drug because it takes time for the antidote to be delivered to the cells needing protection because the delivery is relatively slow. Of course, one can hasten the delivery by increasing the amount of carrier bound-antidote, but economic consideration could limit that approach. If a toxic drug is injected daily, liposomes should, of course, be also given daily, for as long as toxic levels of the drug remain in the blood stream. Even where the toxic drug is given only once, if the dosage is sufficiently high as to maintain a toxic level in the blood stream for a few days, it follows that it would be helpful to administer the liposomes on a daily basis.

Regarding the delivery of antidote via liposomes specifically constructed for non-antibody targeting, the following steps illustrate the method. Small, unilamellar liposomes containing monosodium glutamate, size ranging from 12 to 95 nm (120 to 950 Angstroms) diameter, are made from egg phosphatidylcholine and cholesterol (14 mg and 3,87 mg, respectively) by evaporating the lipids dissolved in 2 ml chloroform onto the inner surface of a 250 ml round-bottom flask, adding an aqueous L-buffer solution containing 32 mg/ml glutamate, vortexing the mixture until lipids are uncoated from the flask, and then sonicating the cloudy mixture in bath type sonicator until clear. A volume of 0,5 ml of this suspension, representing 1,25 micromoles of lipid, sterilized by passage through a 0,22 micron bacteriological filter, is injected intravenously to a 100 gram rat 24 & 2 hours before and 24,48,72, and 96 hours after an intraperitoneal injection of vinblastine at the dose of 0,75 mg/kg. Although animals given only vinblastine experience weight loss (from gastrointestinal toxicity of anorexia and diarrhea) and substantial bone marrow destruction, animals given above described liposomes show much abatement of weight loss and marrow suppression.

Other examples include substituting the above drugs and their antidotes with other drug-antidote pairs mentioned in this specification.

A further example: my invention can also be used to create disease models in organisms by allowing a cytotoxic drug to kill are make sick certain body cells while protecting other body cells that can be affected by the cytotoxic agent. For example, one can use FUdR to damage colonic mucosal cells to simulate colitis while protecting small intestine, stomach, esophageal, oropharyngeal, bone marrow, and basal keratinocyte cells from the cytotoxic action of FUdR by the preferential delivery to the latter cells of thymidine using the methods described.

## Claims

1. A composition for reducing undesired effects of a drug upon a body cell, which has been unintentionally affected by said drug and/or which is not the intended target of said drug, said composition consisting of unilamellar liposomes made of lipids whose solid to liquid-crystalline transition temperature is below the body temperature and having a diameter in the range of 12 to 95 nm, and a pre-inducer chemical for an antidote or an antidote for said drug, said preinducer or said antidote being capable of being carried by that small unilamellar liposome.

2. A composition for reducing undesired effects of a drug upon a body cell, which has been unintentionally affected by said drug and/or which is not the intended target of said drug, said composition consisting of unilamellar liposomes substantially made of egg phosphatidylcholine and cholesterol, in the molar ratio of 65:35, respectively and is of 12 to 95 nm (120 to 950 angstroms) in diameter, and a pre-inducer chemical for an antidote or an antidote for said drug, said preinducer or said antidote being capable of being carried by that small unilamellar liposome.

3. The composition of claim 1 or claim 2, wherein the diameter of the liposomes is in the range of about 35 to 60 nm.

4. The composition of any of the claim 1 through 3, wherein said liposome is neutral or positively charged.

5. The composition of any of the claims 1 through 4, wherein said antidote is one selected of the group comprising:
glutamate, folinic acid, deoxyguanosine, thymidine, deoxycytidine, oxypurinol, tryptophan, uridine, aspartic acid, bleomycin hydrolase, xanthine oxidase, a free radical scavanger, or a fragment of deoxynucleic acid.

6. The composition of any of the claims 1 through 5, wherein said antidote is a thiol-containing chemical capable of counteracting said drug effect of a platinum drug.

7. The composition of any of the preceding claims, wherein said antidote is metallothionein.

8. The composition of any of the preceding claims, wherein said pre-inducer chemical is bismuth subnitrate.

9. The composition of any of the claims 1 through 7 for reducing the undesired effect of granulocytopenia.

10. The composition of any of the claims 1 through 8 for use in the cancer therapy.

## Patentansprüche

1. Zusammensetzung zur Verminderung unerwünschter Wirkungen eines Arzneimittels auf eine Körperzelle, die unabsichtlich von dem Arzneimittel beeinflußt worden ist und/oder die nicht das beabsichtigte Ziel des Arzneimittels ist, wobei die Zusammensetzung aus monolamellaren Liposomen besteht, die aus Lipiden hergestellt sind, deren Kristallübergangstemperatur von fest zu flüssig unterhalb der Körpertemperatur liegt und die einen Durchmesser im Bereich von 12 bis 95 nm aufweisen und eine prä-Induktor-Chemikalie für ein Gegengift oder ein Gegengift für das Arzneimittel, wobei der prä-Induktor oder das Gegengift in der Lage sind, von dem kleinen monolamellaren Liposom getragen zu werden.

2. Zusammensetzung zur Verminderung unerwünschter Wirkungen eines Arzneimittels auf eine Körpeizelle, die unabsichtlich von dem Arzneimittel beeinflußt worden ist und/oder die nicht das beabsichtigte Ziel des Arzneimittels ist, wobei die Zusammensetzung aus monolamellaren Liposomen besteht, die im wesentlichen aus Phosphat idylchol in aus El und Cholester in im molaren Verhältnis von jeweils 65:35 hergestellt sind und Durchmesser von 12 bis 95 nm (120 bis 950 Angström) haben und eine prä-Induktor-Chemikalie für ein Gegengift oder ein Gegengift für das Arzneimittel, wobei der prä-Induktor oder das Gegengift in der Lage sind, von dem kleinen monolamellaren Liposom getragen zu werden.

3. Zusammensetzung gemäß Anspruch 2 oder Anspruch 3, bei der der Durchmesser der Liposomen im Bereich von etwa 35 bis 60 nm beträgt.

4. Zusammensetzung gemäß einem der Ansprüche 1 bis 3, bei der das Liposom neutral oder positiv geladen ist.

5. Zusammensetzung gemäß einem der Ansprüche 1 bis 4, bei der das Gegengift eines ist, das aus der Gruppe ausgewählt ist, die umfaßt: Glutamat, Folinsäure, Desoxyguanosin, Thymidin, Desoxycytidin, Oxypurinol, Tryptophan, Uridin, Asparaginsäure, Bleomycin-Hydrolase, Xanthin-Oxidase, einen Fänger freier Radikale oder ein Fragment von Desoxynucleinsäure.

6. Zusammensetzung gemäß einem der Ansprüche 1 bis 5, bei der das Gegengift eine Thiol-enthaltende Chemikalie ist, die der Arzneimittelwirkung eines Platin-Arzneimittels entgegenwirken kann.

7. Zusammensetzung gemäß einem der voranstehenden Ansprüche, bei der das Gegengift Metallothlonein ist.

8. Zusammensetzung gemäß einem der voranstehenden Ansprüche, bei der die prä-Induktor-Chemikalie Bismutoxidnitrat ist.

9. Zusammensetzung gemäß einem der Ansprüche 1 bis 7 zur Verminderung der unerwünschten Wirkung der Granulozytopenie.

10. Zusammensetzung gemäß einem der Ansprüche 1 bis 8 zur Verwendung in der Krebstherapie.

## Revendications

1. Composition permettant de réduire les effets indésirables d'un médicament sur une cellule du corps qui a été affectée accidentellement par ledit médicament et/ou qui n'est pas la cible visée dudit médicament, ladite composition étant constituée de liposomes unilamellaires faits de lipides dont la température de transition de la phase solide à la phase cristal liquide est inférieure à la température du corps et ayant un diamètre dans la gamme de 12 à 95 nm, et d'un produit chimique pré-inducteur pour un antidote ou un antidote dudit médicament, ledit pré-inducteur ou ledit antidote étant capable d'être porté par un petit liposome unilamellaire.

2. Composition permettant de réduire les effets indésirables d'un médicament sur une cellule du corps qui a été affectée accidentellement par ledit médicament et/ou qui n'est pas la cible visée dudit médicament, ladite composition étant constituée de liposomes unilamellaires faits substantiellement de phosphatidylcholine d'oeuf et de cholestérol, dans le rapport molaire de 65/35, respectivement, et de 12 à 95 nm (120 à 950 angströms) de diamètre, et d'un produit chimique pré-inducteur pour un antidote ou un antidote dudit médicament, ledit pré-inducteur ou ledit antidote étant capable d'être porté par ce petit liposome unilamellaire.

3. Composition selon la revendication 1 ou la revendication 2, dans laquelle le diamètre des liposomes est dans la gamme d'environ 35 à 60 nm.

4. Composition selon l'une quelconque des revendications 1 à 3, dans laquelle ledit liposome est neutre ou chargé positivement.

5. Composition selon l'une quelconque des revendications I à 4, dans laquelle ledit antidote est un antidote choisi dans le groupe constitué par : le glutamate, l'acide folinique, la désoxyguanosine, la thymidine, la désoxycytidine, l'oxypurinol, le tryptophane, l'uridine, l'acide aspartique, la bléomycine hydrolase, la xanthine oxydase, un piégeur de radicaux libres, ou un fragment d'acide désoxynucléique.

6. Composition selon l'une quelconque des revendications 1 à 5, dans laquelle ledit antidote est un produit chimique contenant un thiol capable de contrecarrer ledit effet pharmacologique d'un médicament à base de platine.

7. Composition selon l'une quelconque des revendications précédentes, dans laquelle ledit antidote est la métallothionéine.

8. Composition selon l'une quelconque des revendications précédentes, dans laquelle ledit produit chimique pré-inducteur est le sous-nitrate de bismuth.

9. Composition selon l'une quelconque des revendications 1 à 7 permettant de réduire l'effet indésirable de la granulocytopénie.

10. Composition selon l'une quelconque des revendications 1 à 8 à utiliser dans le traitement du cancer.
